# EUROPEAN PATENT APPLICATION

(11) **EP 3 412 199 A1**
(43) Date of publication of application: **12.12.2018**
(21) Application number: 17175038.3
(22) Date of filing: 08.06.2017
(51) Int. Cl.: A61B 5/00, A61B 5/07, H04B 13/00, A61B 1/00, A61B 1/04

(54) **WEARABLE OR IMPLANTABLE SENSOR OR ACTUATOR DEVICE**

(71) Applicant: Koninklijke Philips N.V., 5656 AE Eindhoven (NL)
(72) Inventor: VISWESWARA, Ashoka Sathanur, 5656 AE Eindhoven (NL); MEFTAH, Mohammed, 5656 AE Eindhoven (NL); JOHNSON, Mark Thomas, 5656 AE Eindhoven (NL)
(74) Representative: de Haan, Poul Erik

(57) **Abstract**

A battery-operated sensor device has a sensor circuit with a dormant mode and an active mode. A self-powered wake up circuit uses power harvesting for generating a wake up signal for switching the sensor circuit from the dormant mode to the active mode. In this way, the dormant mode may not have any live circuits draining power. The invention is of interest for any low power sensor where it is desired not to have to tamper with the sensor for prolonged periods (i.e. to change the battery). This is for example of interest for implantable sensor devices.

## Description

### FIELD OF THE INVENTION

This invention relates to sensor or actuator devices such as medical sensor inserts or implants, or wearable medical sensors.

### BACKGROUND OF THE INVENTION

Healthcare costs continue to rise due to the rapidly growing aging population and an increasing number of people with chronic diseases. Healthcare companies recognize this trend and are developing cost-effective medical wearable solutions to address this need.

For example, the applicant has developed a wearable biosensor patch which enables continuous home monitoring of vital signs such as ECG, heart rate, respiration rate, skin temperature and activity. The clinical data is sent to and processed on a cloud-based platform and finally supports the healthcare professionals in their clinical decision making. The biosensor patch is able to prevent hospital readmissions by detecting health deterioration in the home setting as early as possible.

Besides stick-to-skin patches there are other solutions available on the market such as health watches, monitoring belts or straps and health monitoring clothing.

Although the above mentioned wearable monitoring solutions have clear advantages and added value they are still not optimal. It is envisioned that the next generation of wearable devices will be either temporary tattoos (in the form of extremely unobtrusive, ultra-thin, skin contour/topography conforming films adhering to the skin by van der Waals forces) or permanent electronic implantable devices which are placed under the skin.

Implantable devices are seen as an unobtrusive monitoring solution for chronically ill patients. After implantation, they can remain in place for years. During that time, they are unnoticed by the user and provide more ecofriendly alternatives to disposables. Implantable devices are in particular the ultimate solution for chronically ill (elderly) patients, as no additional effort is required by both caregivers and patients. Additionally, these devices can perform some measurements which are not possible on-body.

There are various requirements and challenges for any wearable or implantable monitoring device. These include low power/energy consumption, high comfort, reliable and accurate operation during use, and high signal quality.

This invention relates to the issue that the power consumption should be kept as low as possible, particularly for an implantable (i.e. in-body) device which is intended to be left in situ for prolonged periods, although the same issue is also of general relevance to on-body or near-body devices. The invention is of particular interest for sensing devices, but it may equally be applied to actuator devices such as neurostimulators.

Indeed, low power design has taken an unprecedented importance in current electronics systems design generally. This is due to the fact that more and more applications are battery powered, require long battery time operation and the electronic systems are becoming increasingly miniaturized. Power management of a complex electronics system is typically achieved by shutting down parts of the system or entire system blocks when they are not active. This minimizes the shut-down leakage current substantially thereby reducing the overall power consumption of the system.

However, a device which is designed to respond to an external stimulus (such as a command to perform a sensing function or deliver an actuation) typically has at least one circuit block (known as an always-on circuit) which remains live at all times and hence gives rise to undesired power consumption. Thus, there is still a need to reduce power consumption so as to optimize electrical power and energy management.

### SUMMARY OF THE INVENTION

The invention is defined by the claims.

Examples in accordance with the invention provide a sensor or actuator device, comprising:
a sensor or actuator circuit having a dormant mode and an active mode;
a battery for powering the sensor or actuator circuit;
a wake up circuit for generating a wake up signal for switching the sensor or actuator circuit from the dormant mode to the active mode; and
a self-powered power harvesting circuit for generating the power for operating the wake up circuit from an externally received wireless signal.

This device is able to have a very low power dormant mode because it does not need to maintain a standby sensing mode or to be able to receive external instructions or commands during the dormant mode. Instead, a wake up signal is generated without needing power from the sensor or actuator circuit battery, so that the sensor or actuator circuit can be fully shut down, for example with its battery fully disconnected from the sensor or actuator circuit. The wake up circuit may draw no power from the battery at all. This means that in the dormant mode, there may be no current drawn from the battery (other than leakage currents). Even leakage currents can be eliminated by using advance shutdown technologies such as a MEMS based power switches, which physically disconnect the battery from the sensor system achieving zero power consumption.

The wake up circuit is then able to reconnect the battery to the circuit to enable sensor or actuator functionality to be resumed. This is in response to an external wake up command which is generated for the purpose of waking the sensor or actuator so that it may perform sensor measurements or actuation functions.

This device is of particular interest for an implantable medical sensor or actuator. It enables the battery lifetime to be extended since power is only drawn when sensing or actuation is required.

The sensor or actuator circuit is preferably adapted to remain in the active mode only while the wake up signal is present. The wake up signal may remain present only when the externally received wireless signal is present, or else the wake up circuit may store energy in a capacitor so that the wake up signal lasts longer than the externally received wireless signal. However, the active mode is only enabled by the externally received wireless signal. Thus, the sensor/actuator will only function as long as the external device sending the power to the wake up circuit is active. When the energy source stops (or internal energy in the wake up circuit has dissipated), the wake up signal goes low and the sensor or actuator is shut off.

The power harvesting circuit may comprise an RF energy harvesting circuit. The harvesting of energy from RF signals is well known.

The power harvesting circuit may be adapted to harvest energy from a 10 MHz to 200 MHz RF signal. This signal frequency range corresponds to the frequency used for body channel sensing, for which the body behaves as a waveguide. Note that the term "10 MHz to 200 MHz RF signal" means that the frequency used is somewhere within that range.

The power harvesting circuit may comprise a band pass filter. This means that the sensor or actuator device and its wake up circuit may be responsive to a specific RF signal. Thus, there maybe multiple sensors or actuators, and each one may respond to its own external signal to perform the wake up function.

The power harvesting circuit may instead comprise an optical energy harvesting circuit adapted to harvest energy from an optical wake up command signal. This is an alternative way to generate the wake up signal. An optical signal may be used even for an implanted device depending on the depth of the implant. However, the invention is also of interest for non-implanted devices, in particular for devices which are intended to be left them in place for as long as possible without the power supply becoming exhausted.

The power harvesting circuit may comprise a coded light filter. In other words, coded light may be used to send a wake up command to a specific sensor or actuator device with a network of sensor or actuator devices. By a "coded light filter" is meant a circuit which responds to a specific coded light signal or instruction.

The wake up circuit may comprise a switch circuit in series with the sensor or actuator circuit, both between terminals of the battery. Thus, it provides full disconnection of the power from the sensor or actuator circuit so that there is no standby mode or periodic listening mode of the sensor or actuator circuit.

Almost no leakage current can then be achieved.

The invention also provides a sensor or actuator system, comprising:
a sensor or actuator device as defined above; and
a control device for providing a wake up command to the sensor or actuator device thereby to trigger the sensor or actuator device to switch to its active mode.

The sensor or actuator device is for example an implantable device and the control device is a non-implantable device. The control device for example comprises a signal generator having an output frequency in the range 10 MHz to 200 MHz and electrodes for application to the skin. Alternatively, the control device may comprise an optical signal generator.

The system may further comprise a camera system for monitoring vital signs, and for operating the control device in dependence on the monitored vital signs. In this way, a camera system is used to determine when the sensor signals should be obtained.

Examples in accordance with another aspect of the invention provide a method of operating a sensor or actuator device, comprising:
placing a sensor or actuator circuit into a dormant mode;
harvesting power from an externally received wireless wake up command;
locally at the sensor or actuator circuit, generating a wake up signal for switching the sensor or actuator circuit from the dormant mode to an active mode using the harvested power; and
obtaining a sensor reading or performing an actuation using power from a local battery of the sensor circuit.

The sensor or actuator circuit may remain in the active mode only while the wake up signal is present.

The sensor or actuator circuit maybe part of an implantable device and the externally received wireless wake up command may be generated by a non-implantable device, wherein the externally received wireless wake up command is an RF signal in the 10 MHz to 200 MHz range or an optical signal.

### BRIEF DESCRIPTION OF THE DRAWINGS

Examples of the invention will now be described in detail with reference to the accompanying drawings, in which:
Figure 1 shows in schematic form an example of a system in accordance with an example;
Figure 2 shows a first human body model for signal transmission inside the body;
Figure 3 shows a second human body model for signal transmission inside the body;
Figure 4 shows a plot of human body transmission characteristics versus frequency;
Figure 5 shows a typical known architecture of a system with a dormant mode and an active mode;
Figure 6 shows a sensor device in accordance with the invention in more detail;
Figure 7 shows an example of the control device architecture in more detail;
Figure 8 shows an example of the self-harvesting unit of the sensor device in more detail;
Figure 9 shows another example of a system in accordance with the invention; and
Figure 10 shows a sensor control method.

### DETAILED DESCRIPTION OF THE EMBODIMENTS

The invention provides a battery-operated sensor device having a sensor circuit with a dormant mode and an active mode. A self-powered wake up circuit uses power harvesting for generating a wake up signal for switching the sensor circuit from the dormant mode to the active mode. In this way, the dormant mode may not have any live circuits draining power. The invention is of interest for any low power sensor where it is desired not to have to tamper with the sensor for prolonged periods (i.e. to change the battery). This is for example of interest for implantable devices. This term is used to indicate any electronic device for insertion into the body.

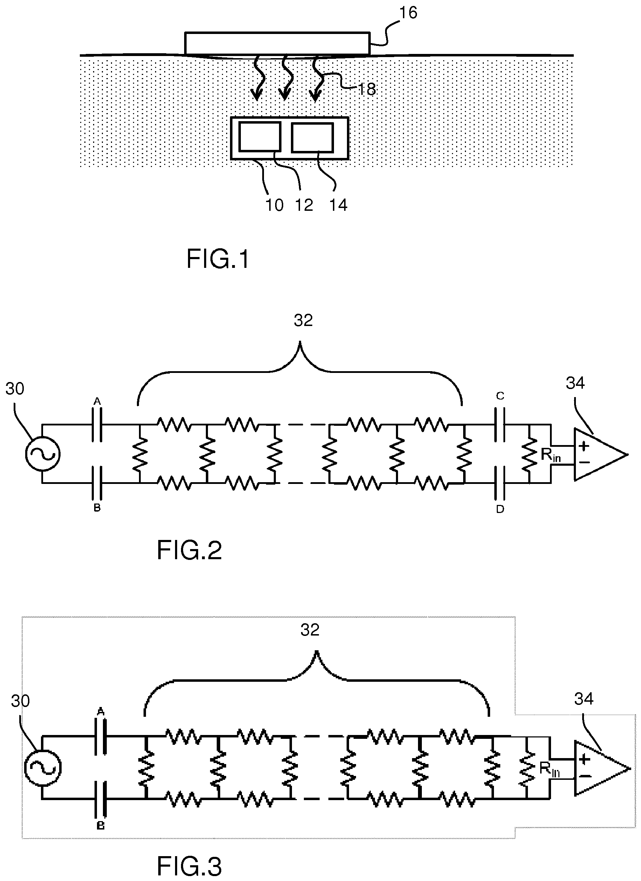

Figure 1 shows the general configuration of the invention based on the example of a system with an implanted sensor.

The system comprises an implanted sensor device 10 which comprises a battery-operated sensor circuit 12 and a self-powered power harvesting circuit 14. For a more general application of the invention, the device may comprise a sensor or an actuator, hence either for monitoring physiological parameters or for delivering a treatment.

A control device 16 is used to generate a wake up command 18 in the form of a wireless command transmitted to the sensor device 10. The power harvesting circuit generates power from the wireless command, which is used to operate a wake up circuit (which is part of the sensor device). The wake up circuit is able to switch the sensor circuit (i.e. the remainder of the sensor device) from a dormant mode to an active sensing mode by generating a wake up signal.

In a preferred example, the active mode remains in place only while the wake up signal is present, so that no timing routines are required in the sensor device. The wake up signal may remain present only when the wireless command is actually present, or else the wake up circuit may store energy in a capacitor so that the wake up signal lasts longer than the externally received wireless command. However, the active sensing mode is only enabled by the externally received wireless command.

In the particular case of an implanted device, the wake up generated by the control device 16 needs to be in the form of a signal which can be transmitted through body tissue.

There have been several academic works which describe an electrical field model of the human body. The model shows the frequency response of the human body as a signal transmission medium. It is measured by capacitive coupling of an electrical signal of certain frequency and amplitude at one point on the human body. The received signal measured at a different point on the body by a sensor is analyzed for various transmitter frequencies and various distances (and body locations) between the transmitter and the sensor.

In the article of N Namjum Cho, "The Human Body Characteristics as a Signal Transmission Medium for Intrabody Communication", IEEE Transactions on Microwave Theory and Techniques, 2007, the human body characteristics as a signal transmission medium is presented. It suggests a near-field coupling model of the human body by modeling the human body into three cylinders, two for the arms and one for the human torso. The arms and the torso are then modeled as distributed RC network.

From measurements taken, a linear relationship in the attenuation of the signal from a transmitter towards a receiver is observed as the receiver is placed at varying distances from the transmitter, in particular when the frequency used is in a preferred range of 10 MHz to 200 MHz. At a frequency lower than 10 MHz, the signal wavelength is much larger than the size of the human body and the electric field around the human body is almost uniform (though varying in time), which means its phase has almost the same value everywhere on the body at some point in time. In this condition, the time-varying electric field around the human body can be regarded as a quasi-static field.

On the other hand, at frequencies greater than 10 MHz and up to 200 MHz, the body behaves like a waveguide for the RF signal at such frequencies, since the signal wavelength is comparable to the human body dimensions.

Figure 2 shows a human body model for signal transmission inside the body for such body channel sensing signal frequencies. A signal source is shown as 30 with two electrodes, which couple through capacitances to the conductive body tissue 32, which is represented by a ladder network of resistances. There is capacitive coupling to a receiver amplifier 34. Figure 2 is a model where the transmitter and the receiver are located on the surface of the human body.

In the model in Figure 2, the capacitors A and B at the signal source denote the capacitance formed between the two electrodes of a transmitter and the conductive tissues beneath the epidermis. The capacitors C and D at the receiver side denote the capacitances formed between the receiver electrodes and the conductive tissues. This model for example may represent a power source capacitively coupled to the human body and an electrical tattoo placed on the human body at an adjacent location. The capacitors at the signal source form a high-pass filter with the input resistance of the conductive tissues and another high-pass filter is formed between the input resistance of the receiver and the capacitors at the receiver side. These two high-pass filters for example create a 40dB/decade roll-off of the input signal applied from the transmitter.

Figure 3 shows a model where the transmitter is located outside the human body but the receiver is located inside the body. In Figure 3, the signal source 30 again couples to the conductive body tissue through capacitances (capacitors A and B) but the receiver connects through resistors.

This model for example may represent a power source capacitively coupled to the human body and an implanted device placed inside the human body. For this model, there is only one high-pass filter formed at the transmitter side giving a 20dB/decade roll-off.

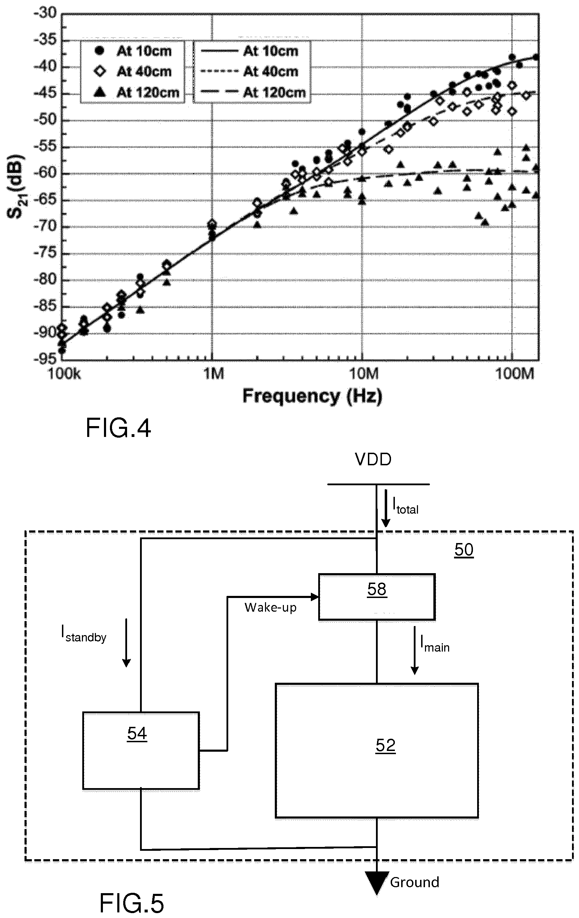

The distributed RC model has been found to be a good fit with measurements, as shown in Figure 4, which shows the S21 insertion loss versus frequency for three different transmission ranges. The discrete points show measured data and the plot lines show simulations.

From the two models and the measurement results, it is clear that the body channel is dominated by the high-pass filter formed by the capacitive coupling to the human body and the spreading resistance of the conductive tissues under the epidermis. Also for lower frequencies the body acts as a single node and this can be seen in no variation of the channel gain for various distances between the transmitter and the receiver. As the frequency of operation increases, the wavelength of the signal becomes comparable or smaller with respect to the size of the human body. As a consequence, from a single node model, there is a move towards a surface wave phenomenon where the channel gain depends on the distance between the transmitter and the receiver.

These studies show that it is possible to use the human body as a medium for transmission of RF signals.

It is well known that to implement ultra-low power wireless sensor networks (WSN), ultra-low power wireless communication capability is needed. Radio transceivers consume power whenever they are active, so it is efficient to leave the receiver off and wake it up asynchronously only when needed. A dedicated wake up receiver can continuously monitor the channel, listening for a wake up signal from other nodes and activating the main receiver upon detection. By maximizing the sleep time of the main receiver without compromising network latency, the use of a wake up receiver can improve overall network performance and reduce the system power consumption. Wake up receivers are applicable in asymmetric links such as "active" RFID, where the tag listens in standby mode until queried by a reader.

Figure 5 shows a typical low power architecture for an electronic system 50 such as a sensor. The system 50 has a main electronics block 52 and a standby block 54. The main electronics block can be isolated from the power supply 56 by a switching circuit 58. The standby block 54 is always on and hence listening (either continuously or periodically) for commands. It generates a wake up signal when requested, which enables the supply of power to the main electronics block 52. The energy consumption of the standby block (and hence the current drawn I_{standby}) is very small compared to the main electronics in active mode (an hence the current drawn Iₘₐᵢₙ) so it helps to reduce energy expenditure of the main electronics in shut-down mode. Thus, the total current Iₜₒₜₐₗ when in active mode is dominated by the main circuitry. However in the case of an implantable device, due to its miniature size, the battery or storage element used is small limiting the energy storage capacity severely.

When the switching circuit 58 is off, the total shut down current contributed by the main electronics block is significantly reduced. However the standby block is always on and has a direct connection to the power supply and thus continues to draw the current I_{standby}. This standby block for example has timing circuitry for a micro controller unit (MCU) and for any communication systems. It is used to generate the suitable wake up signal to wake up the main electronics block in response to an external trigger event. However, the shut-down current contribution of the standby block becomes a significant part of the total shut-down current of the entire system when the electronics system goes into shut-down mode.

This invention aims to reduce or eliminate this standby power consumption and is of interest for severely energy constrained systems such as implantable (or otherwise insertable) devices. The implantable device is only triggered to perform a measurement or an analysis of certain body parameter when triggered by the external wake up command. The wake up command responds to an abnormal physiological reading, and hence decides to instruct a more detailed analysis by triggering the implantable device.

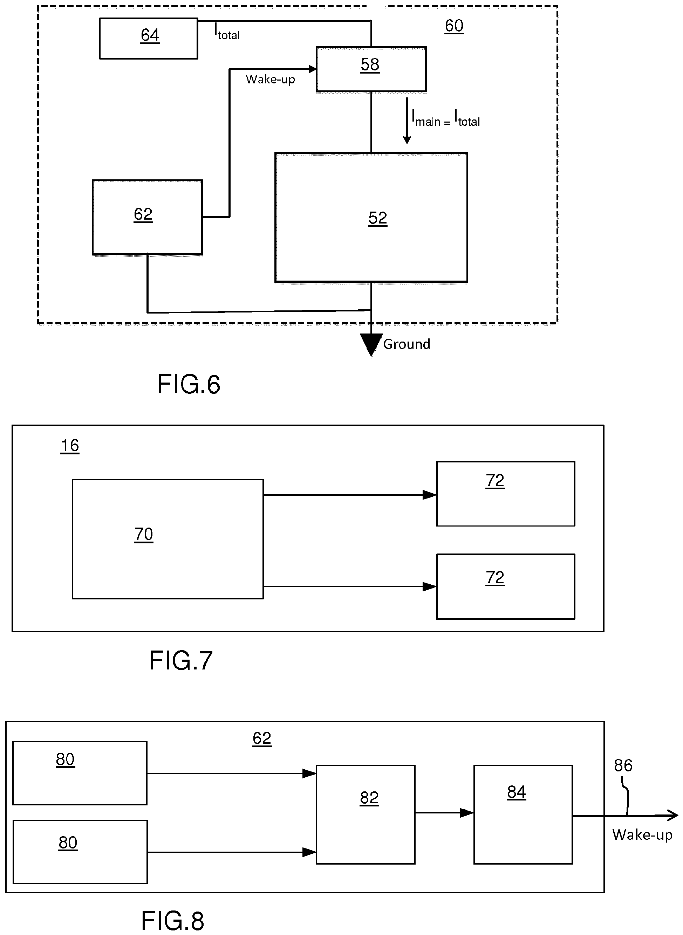

Figure 6 shows the sensor system 60 of the invention, in which the same components as in Figure 5 are given the same reference numbers. The standby unit 54 has been replaced by a self-powered unit 62 for generating a wake up signal to the switch arrangement 58. The battery is also shown as 64.

The unit 62 harvests electrical energy from a wireless signal (such as an RF body channel frequency signal) transmitted by the control device 16 (see Figure 1) which is a wearable or other body worn device. The frequency of the RF signal used is for example such that the human body behaves as a wave-guide for the signal propagation at that frequency. To achieve high specificity for the wake up signal generation, a passive bandpass filter is used at the unit 62 and a single carrier frequency is used for signal transmission.

If multiple sensor devices need to be triggered at different times, different wake up signal generation frequencies can be used so that the sensor device for which the wake up signal is not intended does not harvest enough energy to generate a false wake up signal in a set time frame.

By using a self-powered unit 62 instead of an always-on standby block for wake up signal generation, a significant reduction in shut-down energy consumption is achieved. There is no longer any continuous current drawn during the dormant mode so that the total shut-down current of the system is significantly reduced.

By using body channel propagation methods for wake up signal transmission instead of traditional RF methods, body occlusion to GHz RF signals is avoided, improving the wake up signal generation efficiency. The security of the wake up command generation can also be improved since the transmission of the wake up command is only through the human body, external transmitters outside the body will not wake up and activate the implanted device.

Another disadvantage of using traditional GHz RF signals is that, since the GHz RF signal cannot be directed or constrained in a particular direction or restricted to a specific space due to its transmission properties, the number of false wake ups will become high by using such GHz RF signal for wake up signal generation.

Figure 7 shows the control device 16 having a signal generator 70, and a pair of electrodes 72. The signal generated is for example an RF signal suitable for body channel transmission.

The self-powered unit 62 is shown in more detail in Figure 8. It has a pair of receive electrodes 80 to receive the RF signal coupled by the control device, a bandpass filter 82 and an energy harvesting system 84. The unit 62 generates a wake up signal 86. The band-pass filter allows only the RF signal of a particular frequency to be harvested. In this case the band-pass center frequency is chosen to be the same as the RF signal frequency of the control device.

The energy harvesting circuit 84 for example comprises a receive antenna, a rectifier (diode, diode bridge, or other diode configuration), and other signal processing units for processing the received signal and/or the rectified signal, such as band pass or low pass filters and voltage booster circuits. Impedance matching circuits are also typically used.

Wireless energy harvesting systems are well known, By way of example, a summary is given in "Harvesting Wireless Power" by C. Valenta and G. Durgin, IEEE microwave magazine June 2014.

Depending on the distance between the transmitter and the implantable device, the energy transferred from the control device can vary. For long distances between the external on-body control device and the implantable device, the energy transferred per cycle of the RF wave will be very small due to attenuation of the RF signal along the human body. However for shorter distances, the body attenuation is smaller and hence higher amount of energy can be transferred from the external on-body device to the insert/implant device. Based on the energy received, the time required to generate the wake up signal can vary. So once the implantable device is woken up, it sends an acknowledgement back to the transmitter indicating that the implantable device has been woken up.

The method for indicating the acknowledgement can also be via human body communication technology or via standard wireless communication technologies such as Bluetooth.

If there are multiple implantable devices using the same body channel for generating the wake up signal, then using the same frequency for all the implantable devices will generate too many false wake up events of the devices leading to higher power consumption of the implantable device not intended to be woken-up. To avoid this, the external control device uses separate carrier frequencies each one dedicated to one single implantable device. The implantable device has a bandpass filter with a configurable center frequency which can be set to one carrier frequency during the device configuration step. The carrier frequencies (spacing between different frequencies) are chosen such that the bandpass filter designed for one carrier frequency can sufficiently reject other frequencies thereby limiting false wake up generation.

Besides RF energy (which may be based on WiFi or Bluetooth signals), other energy harvesting principles may be used by the unit 62. For example it may make use of optical (light) energy. The implantable device then comprises a photo-sensitive element (e.g. photo-voltaic cell).

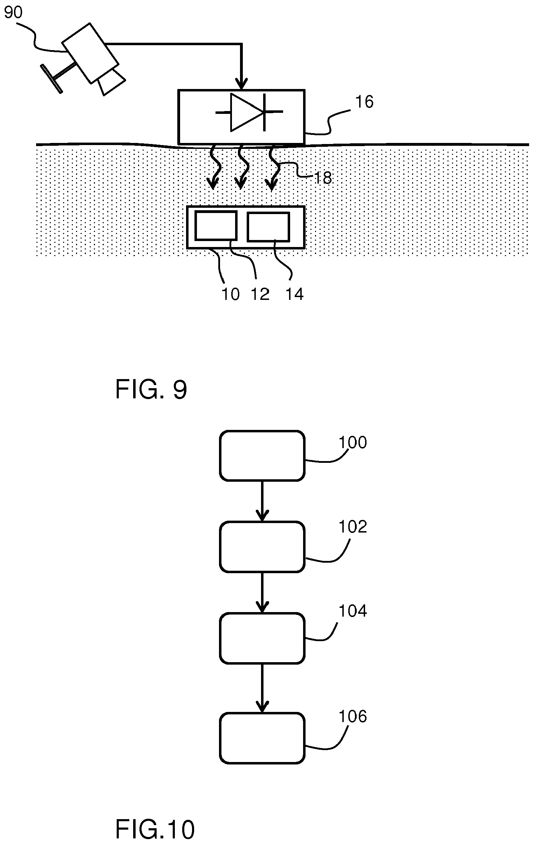

Figure 9 shows an example of the system. The control device 16 comprises a light source. The system may additionally have a vital signs camera 90, which keeps track of the health condition of a patient. As soon as the camera 90 detects an abnormality, dedicated illumination (e.g. light with a unique wavelength, or in the form of coded-light) is emitted towards the implantable device 10 to power the self-powered unit.

False wake ups, when there are multiple implantable devices, can be prevented by techniques such as coded lighting, a unique wavelength per implantable device, etc. Furthermore directional light can be applied, so that light is provided only to the intended implantable device. The camera 90 can, based on body part recognition (face, arm, leg, etc.) also aid in directing the light beam to the right spot on the body. This also prevents activating implantable devices of other patients.

Of course, the use of a camera to detect vital signs is not limited to optical energy harvesting and may be used to trigger an RF activation of the sensor.

Near-infrared (NIR) light may be used because it is not visible to the human eye.

Figure 10 shows a method of operating a sensor device, comprising:
in step 100, placing a sensor circuit into a dormant mode;
in step 102, harvesting power from an externally received wireless wake up command signal;
in step 104, locally at the sensor circuit, generating a wake up signal for switching the sensor circuit from the dormant mode to an active mode using the harvested power; and
in step 106, obtaining a sensor reading using power from a local battery of the sensor circuit.

The invention has been described with reference to an implantable system. However the invention can also be applied to wearable devices. The invention enables the battery life to be extended for all sensor configurations. For an implanted device, body channel RF signals are of particular interest for the energy harvesting. However, other wireless energy harvesting approaches may be used such as capacitive coupling methods and inductive coupling methods as well as the optical approach described above.

The energy harvesting is used to generate the wake up signal, and this may be the only use to which the harvested energy is put. However, an alternative is that energy is harvested for recharging the sensor device battery if a rechargeable battery is used. This then combines an extremely low power dormant mode as well as a capability for remote wireless battery recharging to extend further the lifetime of the battery of the sensor device.

The sensor device may respond to the wake up signal in any known manner.

In the preferred example as explained above, the sensor or actuator device only remains active while the wake up signal is present. The sensor signals are then directly read out, and when the sensor signals have been received, the externally generated wireless command is ended. This has the advantage that power can be fully removed from the sensor or actuator device as soon as the switch 58 is no longer closed. The switch is basically directly controlled by the wake up signal.

However, the sensor device may instead be triggered to take a single reading or a set of readings, or it may remain active for a prolonged (predefined) sensing period during which sensor measurements are taken periodically. This however requires the switch 58 to remain closed even after the wake up signal has ended, so requiring a more complicated switch circuit 58 and timed power shut down mechanism.

The sensor device may transmit the sensor signals immediately and/or it may store them in a local memory for subsequent download to an external device. A wireless communications system is provided for this purpose. The invention does not concern the sensing functionality as such and many possibilities will be known to those skilled in the art.

As mentioned above, the invention may be applied to sensor or actuator devices. Sensor devices are for example used for monitoring vital signs such as heart rate, blood pressure, respiration rate etc. Actuators may be used for controlling nerve or brain response (for example to control pain or seizures) or for controlled drug delivery.

Other variations to the disclosed embodiments can be understood command for example comes from an external wearable device and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A sensor or actuator device (12), comprising:
a sensor or actuator circuit (52) having a dormant mode and an active mode;
a battery (64) for powering the sensor or actuator circuit;
a wake up circuit (62) for generating a wake up signal for switching the sensor or actuator circuit from the dormant mode to the active mode; and
a self-powered power harvesting circuit (62) for generating the power for operating the wake up circuit from an externally received wireless signal.

2. A sensor or actuator device as claimed in claim 1, wherein the sensor or actuator circuit (52) is adapted to remain in the active mode only while the wake up signal is present.

3. A sensor or actuator device as claimed in claim 1 or 2, comprising an implantable medical sensor or actuator.

4. A device as claimed in any preceding claim, wherein the power harvesting circuit (62) comprises an RF energy harvesting circuit, for example adapted to harvest energy from a 10 MHz to 200 MHz RF signal.

5. A device as claimed in claim 4, wherein the power harvesting circuit comprises a band pass filter.

6. A device as claimed in claim 1, 2 or 3, wherein the power harvesting circuit (62) comprises an optical energy harvesting circuit adapted to harvest energy from an optical signal.

7. A device as claimed in claim 6, wherein the power harvesting circuit comprises a coded light filter.

8. A device as claimed in any preceding claim, wherein the wake up circuit comprises a switch circuit (58) in series with the sensor or actuator circuit (52), both between terminals of the battery.

9. A sensor or actuator system, comprising:
a sensor or actuator device as claimed in any preceding claim; and
a control device (16) for providing a wake up command to the sensor or actuator device thereby to trigger the sensor or actuator device to switch to its active mode.

10. A system as claimed in claim 9, wherein the sensor or actuator device (12) is an implantable device and the control device (16) is a non-implantable device.

11. A system as claimed in claim 10, wherein the control device (16) comprises a signal generator having an output frequency in the range 10 MHz to 200 MHz and electrodes for application to the skin.

12. A system as claimed in claim 9 or 10, wherein the control device (16) comprises an optical signal generator.

13. A system as claimed in any one of claims 9 to 12, further comprising a camera system (90) for monitoring vital signs, and for operating the control device in dependence on the monitored vital signs.

14. A method of operating a sensor or actuator device, comprising:
(100) placing a sensor or actuator circuit into a dormant mode;
(102) harvesting power from an externally received wireless wake up command;
(104) locally at the sensor or actuator circuit, generating a wake up signal for switching the sensor or actuator circuit from the dormant mode to an active mode using the harvested power; and
(106) obtaining a sensor reading or performing an actuation using power from a local battery of the sensor circuit.

15. A method as claimed in claim 14, wherein the sensor or actuator circuit remains in the active mode only while the wake up signal is present.
